# EUROPEAN PATENT APPLICATION

(11) **EP 0 943 301 A1**
(43) Date of publication of application: **22.09.1999**
(21) Application number: 99104864.6
(22) Date of filing: 11.03.1999
(51) Int. Cl.: A61F 2/06

(54) **A mesh structured expandable stent**

(30) Priority: 18.03.1998 IT MI980549
(71) Applicant: Medical Technology S.p.A., 22060 Novedrate (CO) (IT)
(72) Inventor: Greco, Francesco, c/o Medical Technology S.p.A., 22060 Novedrate (CO) (IT)
(74) Representative: Ferraiolo, Ruggero

(57) **Abstract**

Meshes formed by elements (MA) that are sensibly U shaped and specular to one another, coaxial and with the open parts facing one another, are transversally joined in order to form associations of meshes having at least one intra-mesh connecting segment (C1) and that are longitudinally associated, joined by a inter-mesh connecting segment (C2), in order that the stent expands and elongates under the effect of a vessel-surgery technique balloon.

## Description

The present invention concerns a mesh structured expandable stent, where stent is intended as a prosthesis suited to preserving the passageway in a tubular part of a human body, such as an artery or a biliary duct.

The prior art comprises expandable stents realized with a mesh structure that allows to position the stent on site retracted in the radial sense and that is then made to expand either spontaneously or by means of the "balloon" technique in vessel-plastic surgery.

Generally these stents are made of special metal alloys or also with appropriate materials, such as polymers.

The drawback of the known expandable stents lies in the fact that the expansion is accompanied by a retraction in the longitudinal direction and this may take place also asymmetrically with the obvious consequence that the stent may not entirely cover the damaged part of the tube in which it is applied.

In a previous patent application (IT - MI97A002495) the applicant disclosed an expandable stent able to elongate while it expands radially into the desired position.

The structure of the expandable stent of the present application represents a variation to the structure previously invented by the applicant, compared to which, in addition to maintaining the advantage of elongating while the "balloon" expands it in the location planned, also presents the advantage in that, given an equal weight of the structure, it expands more and is of considerably simpler construction.

The following description employs conventional terms, that are defined below, adopted in order to simplify the description of the structure invented and the claims.

"Mesh" is the name of an element of the structure made up of two specular coaxial U shapes with their open parts facing one another.

Each of these shapes that make up the mesh have a closed part, conventionally known as "mesh vertex" and two loose ends known as "loose ends of the mesh".

It is understood that said U shape may have a number of variations to realize the same solution, one of these variations being the V shape.

"First association of meshes" is the name given to an element made up of a number of meshes transversally aligned and connected to one another continuously in correspondence of their loose ends. Moreover, in the structure described there is a connecting segment that will conventionally be called "intra-mesh connecting segment". Said element joins together two vertexes of the same mesh lengthways in order to form a connection inside the same mesh to form at least one of first association of meshes that will form the stent.

Said first association of meshes is associated longitudinally to identical associations so as to form what is conventionally called "second association of meshes". Said unit is made up of at least two first associations of mesh coaxially connected by means of a connecting segment conventionally called "inter-mesh connecting segment" that longitudinally joins two meshes aligned in correspondence with their free ends in at least one of the second associations of meshes.

This closed cylindrical structure, when subjected to internal pressure by a balloon, expands radially owing to the loose ends of the mesh aligned transversally extend and the vertexes of said meshes spacerun from one another,while the structure expands longitudinally owing to the vertexes of the coaxial meshes spacerun from one another.

A type of structure such as that described features advantages in its simple construction and in its increased ability to expand.

The invention is illustrated below in further detail with the support of drawings in which:
- Fig. 1 is a lateral view,
- Fig. 2 is a first detail from Fig. 1,
- Fig. 3 is a second detail from Fig. 1, and
- Fig. 4 is a detail from a second lateral view.

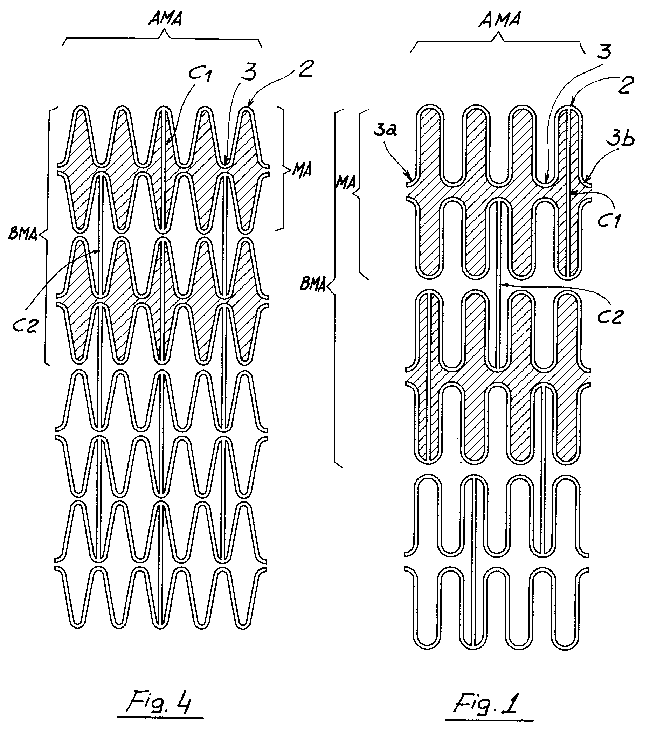

Fig. 1 shows a detail of a plane development of a stent in which the single meshes MA (there are 12) are shown, the first associations between them AMA (there are 3 made up of 4 meshes each) two of which have been cross-hatched for better viewing, and their second associations BMA and the intra-mesh C1 and inter-mesh C2 connecting segments (there are an overall 5, of which 3 are intra-mesh and 2 are inter-mesh). Each AMA has an intra-mesh connecting segment C1 that joins vertexes 2 of the elements of a single mesh MA and is longitudinally associated to other first associations AMA by means of an inter-mesh C2 segment that joins the loose ends 3 of meshes aligned so as to realize the connection between meshes lengthways.

Said intra and inter mesh connecting segments may be conveniently distributed alternately in the stent structure, such as for example an intra-mesh connection every four meshes and one inter-mesh connection every first association of meshes.

By observing Figure 1, the function of said segments C1 and C2 is understood as being that of keeping together the meshes in their first and second associations AMA and BMA and that of providing solidity to the resulting cylindrical structure, being sufficient for the purpose because the ends 3a and 3b will be welded into the resulting stent. The figure also shows how the distribution of segments C1 and C2 in the structure of the stent is obtained by conveniently alternating intra-mesh C1 segments and inter-mesh C2 segments.

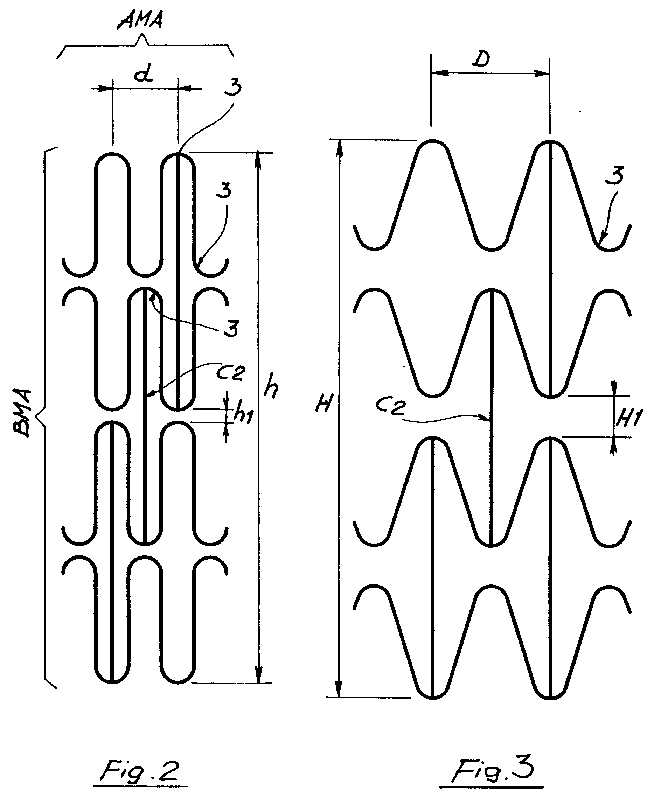

Figure 2 shows a sketch detail of an association of meshes before the stent is expanded: two meshes of a first association of meshes AMA are joined by means of the loose ends 3 and the distance between their vertexes is shown as d. Said AMA is connected by means of segment C2 to an identical association of coaxial meshes and the distance between the vertexes of said longitudinally aligned mashes is shown as h1. The overall length of the resulting BMA is shown as h.

Figure 3 shows the same association of meshes after the stent has expanded: the loose ends 3 of the two meshes in their first association AMA spacerun from one another and the distance between the vertexes of said meshes is D>d. Also the vertexes 2 of the meshes that are longitudinally aligned in their second association BMA spacerun from one another and the distances between said vertexes is H1>h1. The overall length of BMA is H>h. This proves that the expansion of the stent in the radial direction has also produced an elongation of the stent.

Figure 4 is a detail of the plane development of a stent in which the mesh MA is made up of two V shaped elements that are specular, coaxial and with their open ends facing one another. The stent structure is obtained by means of associations between said meshes in order to form the first and second associations as described in Figure 1.

The figure also shows that there is one intra-mesh connecting segment C1 and two intra-mesh connecting segments C2 every 5 meshes and that the connecting segments C1 and C2 are distributed in the stent structure alternately and aligned, respectively, each along a common axes.

The figures described show how said parts are associated in order to provide a stent structure comprising first associations of meshes that have intra-mesh connecting segments that join the vertexes of two specular U or V shaped elements, and in which each first association of meshes is longitudinally connected to other identical first associations of meshes obtained by means of inter-mesh connecting segments that join the loose ends of meshes that are coaxial to one another and in which said connecting segments are distributed in a conveniently alternated intra and inter mesh way.

It is understood that the dimensions of the single elements will be suited to and consistent with each concerned case and that the stent may comprise a number of AMA elements, made up of one or more MA elements, suited to conferring the required diameter to the stent, that the BMA may include two or more AMA elements according to the length required for the stent, and that the connecting segments C1 and C2 are conveniently distributed within the stent structure, as alternate intra and inter mesh connections, aligned along the same axis or casually arranged.

## Claims

1. A mesh structured expandable stent **characterised** in that the meshes are formed by elements (MA) that are sensibly U shaped and specular to one another, coaxial and with their open parts facing one another, each of these shapes having a vertex (2) and two loose ends (3), and that comprises meshes transversally aligned in correspondence with their loose ends (3) to form first associations of meshes (AMA) with an intra-mesh connecting segment (C1) that joins two vertexes (2) of a same mesh in at least one of the first associations of meshes, and that each of said first associations of meshes is longitudinally connected to other identical associations of meshes to form second associations of meshes (BMA) by means of an inter-mesh connecting segment (C2) that joins the loose ends (3) of meshes coaxial to one another in at least one of the second associations of meshes, each of said first associations of meshes being laterally and continuously connected to other identical first associations in order to form a closed cylindrical structure in which, once it is subjected to internal pressure by a balloon, the vertexes of the meshes that are transversally aligned spacerun from one another and the vertexes of meshes that are longitudinally aligned spacerun from one another thus expanding the stent both longitudinally and radially.

2. A mesh structured expandable stent according to claim 1, characterized in that said intra-mesh (C1) and inter-mesh (C2) connecting segments are alternately distributed in intra and inter-mesh way.

3. A mesh structured expandable stent according to claims 1 and 2, characterized in that said intra-mesh (C1) and inter-mesh (C2) connecting segments are, respectively, aligned each along the same axis.

4. A mesh structured expandable stent according to claim 1, characterized in that said intra-mesh (C1) and inter-mesh (C2) connecting segments are casually distributed.
